# EUROPÄISCHE PATENTSCHRIFT

(11) **EP 1 642 033 B1**
(45) Veröffentlichungstag und Bekanntmachung des Hinweises auf die Patenterteilung: **17.02.2010**
(21) Anmeldenummer: 04740570.9
(22) Anmeldetag: 02.07.2004
(51) Int. Cl.: F04D 29/04, A61M 1/10, F04D 13/06

(54) **ZENTRIFUGAL-PUMPE**
CENTRIFUGAL PUMP
POMPE CENTRIFUGE

(30) Priorität: 04.07.2003 DE 10330434
(43) Veröffentlichungstag der Anmeldung: 05.04.2006
(73) Patentinhaber: Jostra AG, 72145 Hirrlingen (DE)
(72) Erfinder: MENDLER, Nikolaus, 82335 Berg (DE)
(74) Vertreter: Kohler Schmid Möbus
(86) Internationale Anmeldenummer: PCT/EP2004/007211
(87) Internationale Veröffentlichungsnummer: WO 2005/003565

(56) Entgegenhaltungen:
- US-A- 5 158 440
- US-A- 5 470 208
- US-A- 5 685 700

## Beschreibung

Die Erfindung betrifft eine Zentrifugal-Pumpe, insbesondere für Blut in Herzersatz- oder -unterstützungseinrichtungen, nach dem Oberbegriff des Anspruchs 1. Insbesondere betrifft die Erfindung eine elektrisch angetriebene Rotationspumpe vom radialen/zentrifugalen Typ zur permanenten Implantation bei Kranken mit terminaler Insuffizienz des Herzens, die einer mechanischen Unterstützung ihres Blutkreislaufs bedürfen.

An Blutpumpen, insbesondere an Blutpumpen oder an Pumpen für andere empfindliche Flüssigkeiten sind besondere Anforderungen zu stellen:
1. Hoher hydraulischer Wirkungsgrad, um die an das Blut/die Flüssigkeit abgegebene Verlustwärme gering und den Energiespeicher klein zu halten.
2. Vollständig berührungslose Rotation des Rotors innerhalb eines hermetisch dichten Pumpengehäuses, wodurch jeder Verschleiß, Abrieb und lokale Wärmeentwicklung durch mechanische Reibung ausgeschlossen werden.
3. Vermeidung von stehenden Wirbeln und Totwassern sowie minimale Verweilzeiten des Blutes/der Flüssigkeit in der Pumpe, um Flüssigkeitsschädigungen bzw. die Aktivierung er Blutgerinnung zu vermeiden.
4. Bei Blutpumpen Minimierung der zellschädigenden Schubspannungen, denen das Blut bei Passage der Pumpe ausgesetzt ist.
5. Fehlersicherheit durch Elimination komplexer sensor-basierter Lageregelungen des Rotors bei gleichzeitig reduziertem Energieverbrauch.
6. Elimination eines Antriebsmotors mit gelagerter Welle, die einem Verschleiß ausgesetzt ist.

Blutpumpen konventioneller Bauart, bei denen der Antrieb des Rotors durch einen Elektromotor mit gelagerter Welle erfolgt, die das Pumpengehäuse durchdringt und mit einer Wellendichtung versehen ist, sind daher zur permanenten Implantation ungeeignet. Hermetisch dichte Gehäuse, durch deren Wandung der Pumpenrotor mittels einer Magnetkupplung in Drehung versetzt wird, eliminieren zwar Leckagen, benötigen aber immer noch einen externen Elektromotor. Zudem muss der Pumpenrotor im Gehäuse durch von Blut umspülte Spurlager geführt werden, die verschleißen und durch lokale Erwärmung Bluteiweiße denaturieren und das Gerinnungssystem aktivieren können, was zu Embolien durch Abrieb und Gerinnsel führen kann.

Eine vollständig berührungsfreie Rotation des Pumpenläufers im Blut kann durch passive und aktive Magnetlager, hydrodynamische Gleitlager oder eine Kombination dieser Prinzipien erreicht werden.

Jeglicher mögliche Ansatz hierzu hat das Theorem von Earnshaw zu beachten, nach dem es nicht möglich ist, einen Körper durch konstante magnetische, elektrische oder Gravitationsfelder in einer stabilen Lage im Raum schwebend zu halten. Jede scheinbare Gleichgewichtslage ist labil, da sich der Körper hier in einem Maximum potentieller Energie befindet. Es bedarf daher in mindestens einer Raumachse einer von außen auf das System wirkenden stabilisierenden Kraft. Diese muss umso größer sein, je weiter sich der Körper vom Ort des Energiemaximums entfernt. Umgekehrt sind nur kleine Rückstellkräfte erforderlich, wenn sich das System a priori in der Nähe des labilen Gleichgewichts befindet.

Magnetisch gelagerte Pumpenrotoren mit offenen Schaufeln sind in der US 6,227,817 beschrieben. Hier wird eine Kombination von passiven Magnetlagern zur radialen Stabilisierung und sensor-basierter aktiver axialer elektromagnetischer Levitation beschrieben. Neben der aufwändigen Herstellung bedingt diese Lösung eine lang gestreckte Spalte zwischen Rotor und Gehäuse mit nur unzureichender Spülung sowie einen hohen Energieaufwand für die axiale Stabilisierung, die dem erheblichen hydraulischen Axialschub entgegenwirken muss, der von einem offenen Flügelrad erzeugt wird.

Blutpumpen mit vollständiger magnetischer Levitation sind in der EP 0 819 330 B1 und der EP 0 860 046 B1 beschrieben. Hierbei ist der Rotor der Pumpe als Rotor einer permanentmagnetisch erregten elektrischen Synchronmaschine ausgeführt. Das Drehmoment wird durch ein umlaufendes radial angreifendes elektromagnetisches Statorfeld erzeugt, ebenso die Positionskontrolle des Rotors in radialer Richtung. Hierzu dienen separate Steuerwicklungen des Stators, die Signale von Abstandssensoren über elektronische Regelkreise in zentrierende Kräfte umsetzen. Durch die außen liegenden Statoren für Antrieb und Lageregelung benötigt diese Pumpe relativ viel Einbauraum. Die Stabilisierung der drei weiteren, nicht aktiv ansteuerbaren räumlichen Freiheitsgrade erfolgt durch passiv wirkende magnetische Reluktanzkräfte. Es werden außerdem Probleme bei Ausführungen mit offenen Flügelrädern durch den hohen hydrodynamischen Axialschub, der unvermeidlich auftritt, angesprochen. Zur Abhilfe werden zusätzliche aktive oder passive Magnetlager sowie hydrodynamische Hilfsmittel in Form von Düsen, Prallplatten, Zuströmrohren, Fließwiderständen und Dichtungsspalten vorgeschlagen, die allesamt die Komplexität des Systems erhöhen, den Wirkungsgrad verschlechtern, Totwasserzonen schaffen, hohe Schubspannungen induzieren und damit für die Realisierung einer Blutpumpe, insbesondere zur permanenten Implantation völlig ungeeignet sind.

Lagerlose Blutpumpen mit magnetischer Levitation mit offenen Flügelrädern sind außerdem in der US 6,071,093 angegeben. Die Übertragung des Drehmoments erfolgt hier jedoch durch ein axial angreifendes umlaufendes elektromagnetisches Statorfeld. Die axiale Rotorposition und das Verkippen des Rotors im Gehäuse wird durch eine sensor-basierte elektromagnetische Rückkoppelung mittels Aktuatoren stabilisiert, gleichzeitig besorgen passive Permanentmagnetlager die radiale Zentrierung. Die Problematik der axialen Instabilität eines offenen Flügelrades wird - neben einer elektromagnetischen Rückkoppelung mittels Sensoren und Aktuatoren - durch eine fluidisch bewirkte Kompensation gelöst. Diese basiert jeweils auf der Wirkung eines am äußeren Umfang des Rotors angebrachten Drosselspalts, der abhängig von der axialen Rotorposition den Rückstrom auf der schaufelabgewandten Seite des Rotors begrenzt oder freigibt. Auch bei dieser Lösung besteht die Gefahr hoher Schubspannungen und der Erzeugung von Totwassergebieten auf der Rückseite des Rotors.

In der US 5,947,703 ist ebenfalls eine elektromagnetisch levitierte Zentrifugalpumpe beschrieben. Hier erfolgt der Antrieb eines gedeckten Flügelrades mittels einer einseitig axial angreifenden permanentmagnetischen Stirndrehkupplung oder durch ein umlaufendes Statorfeld, deren Attraktionskräfte den Pumpenläufer am Gehäuse anlaufen lassen, wenn nicht durch eine sensor-basierte aktive elektromagnetische Rückkoppelung die axiale Rotorposition geregelt wird. Für den Fall des Versagens dieser Regelung werden mechanische Notlauflager in Form von Spurlagern, Gleitlagern, Punktlagern und hydrodynamischen Drucklagern vorgesehen, die ein lebensbedrohendes Festsetzen des Pumpenrotors verhindern sollen. Gemeinsam ist diesen Vorschlägen der nachteilige mechanische Wandkontakt zwischen Rotor und Gehäuse mit den bekannten Konsequenzen der Blutschädigung.

Die WO 01/42653 A1 beschreibt eine Zentrifugalpumpe mit elektromagnetischer aktiver Lageregelung des Pumpenläufers in allen sechs räumlichen Freiheitsgraden, wobei Position, Geschwindigkeit und Beschleunigung des Rotors nicht durch Sensoren erfasst, sondern aus Stromsignalen der aktiven Magnetlager abgeleitet werden. Dies bedingt nachteilig einen äußerst komplexen mechanischen Aufbau von Rotor und multiplen Statoren sowie eine höchst komplexe Regelelektronik mit zusätzlichem Energiebedarf, zumal zur Vermeidung hoher axialer destabilisierender Kräfte ein eisenloser Motor verwendet werden muss, der sich durch seinen schlechten Wirkungsgrad stark erwärmt.

Die genannten Nachteile aktiver elektromagnetischer Lagerung des Pumpenrotors waren Anlass zu einer Reihe von Erfindungen, bei denen durch eine hydrodynamische Stabilisierung des Rotors/Impellers komplizierte Sensorik und Elektronik eliminiert werden soll.

So werden bspw. in der US 5,324,177 und der WO 01/72351 A2 ein hydrodynamisches Traglager zur radialen Stabilisierung des Rotors einer elektrischen Gleichstrommaschine verwendet, der den offenen Pumpenrotor trägt. Von Nachteil ist hier die lange axiale Erstreckung des engen exzentrischen Lagerspaltes, in dem hohe Schubspannungen wirken, und zu dessen Auswaschung Hilfsschaufeln und ein Spülkreislauf von der Hoch- zur Niederdruckseite der Pumpe erforderlich sind. Diese Anordnung birgt die bekannten Gefahren hoher Scherung und ungenügender Wärmeabfuhr, die zu einer Traumatisierung des Blutes führen.

Teilweise vermieden werden diese Nachteile in der US 6,227,797. Hier ist in einem rotationssymmetrischen Gehäuse der Pumpenläufer so ausgeführt, dass seine Oberflächen allseits gegenüber dem Gehäuse in Richtung der Relativbewegung geneigte Wirkflächen in Form keilförmiger Spalte bilden. Pumpenläufer und Gehäuse bilden somit ein hydrodynamisches dreidimensionales Gleitlager, wie im Maschinenbau durchaus üblich. Der tragende Fluidfilm aus Blut, das als Schmiermittel dieser Keilflächen dient, bedeckt eine große Fläche und ist, besonders am Umfang des Rotors, einer hohen Schubspannung unterworfen, für die typische Werte von 220 N/m² angegeben werden. Diese Scherspannung liegt damit in einem Bereich, in dem eine Schädigung von Blutzellen, insbesondere Thrombozyten, durch Scherung zu befürchten ist. Weitere Nachteile dieser Lösung bestehen darin, dass der offene Pumpenrotor allseits gegenüber dem Gehäuse von engen Spalten umgeben ist, in denen hohe viskose Reibung herrscht. Die Notwendigkeit zur Aufteilung des Rotors in segmentale Blöcke, um den Durchtritt des Blutes vom Einlass zum Auslass der Pumpe zu ermöglichen, steht einer Optimierung der fluidmechanischen Wirksamkeit der Pumpe entgegen. Dementsprechend sind die mitgeteilten hydraulischen Wirkungsgrade mit höchstens 11% prohibitiv niedrig für eine implantierbare Blutpumpe mit erstrebenswert geringem Energiebedarf. Die lange axiale Erstreckung des Rotors bewirkt zudem einen hohen hydrodynamischen Radialschub auf den Rotor, der einen geteilten Spiralkanal nötig machen kann, wodurch die Thrombenbildung begünstigt wird. Außerdem ist das Gehäuse kompliziert herzustellen. Auch die in Abb. 20 dargestellte Ausführungsform eines gedeckten Pumpenrotors mit sektoriell keilförmig strukturierter Oberfläche vermeidet die genannten Nachteile nicht, zumal nicht ersichtlich ist, auf welchem Wege das Blut durch einen derartigen Rotor strömen soll.

Eine ganz ähnliche Lösung einer hydrodynamischen axialen Stabilisierung eines offenen Pumpenrotors durch in Richtung der Rotation geneigte aufschwimmende Keilflächen beschreibt die WO 00/32256. Die Nachteile sind auch hier eine Blutschädigung und eine komplizierte Gehäusekonstruktion. Die radiale Zentrierung des Rotors erfolgt hier außerdem nicht durch hydrodynamische sondern durch permanentmagnetische Reluktanzkräfte einer Stirndrehkupplung oder eines elektromagnetischen Antriebsmotors.

Die WO 99/01663 offenbart einen hydraulisch levitierten Pumpenrotor, der durch archimedischen Auftrieb schwimmen soll, da er dieselbe Dichte wie das zu fördernde Fluid besitzt. Diese Pumpe muss mit zwei Einlässen ausgeführt werden oder der Zustrom muss innerhalb der Pumpe um 180° umgelenkt werden, was große benetzte innere Oberflächen sowie eine fragliche hydrodynamische Stabilität zufolge hat.

Die WO 01/70300 schlägt zur hydrodynamischen Stabilisierung einen kegelförmigen Rotor mit schlitzförmigen Öffnungen zum Strömungsdurchtritt und Leitflächen vor, durch die ein gegen das Gehäuse gerichteter Fluidstrom erzeugt wird, der stabilisierend wirken soll. Sofern dies nicht ausreicht, ist ein aktives Magnetlager zur radialen Stabilisierung vorgesehen, was jedoch einen zusätzlichen elektronischen Aufwand bedeutet. In einer Reihe von Patenten (WO 00/32257, WO 00/64508, EP 1 027 898 A1, US 5,840,070) werden Kombinationen der unterschiedlichsten Prinzipien zur Stabilisierung des Pumpenläufers verwendet: Kugel-Spurlager, passive permanentmagnetische Radiallager, aktiv sensorbasierte elektromagnetische Axiallager, hydrodynamische Keilflächenlager sowohl mit axialer als auch radialer Wirkung, ergänzt durch Hilfskonstrukte wie Profilierungen des Rotors und/oder des Gehäuses durch Auflagen, Rippen, Endscheiben, Kanäle und anderes.

Bemerkenswert ist, dass stets mindestens drei dieser Prinzipien in Kombination verwendet werden müssen, um eine berührungslose Rotation des Laufrades in der Pumpe zu gewährleisten und dass in den Keilflächenlagern bei den angegebenen Spaltweiten von ca. 0,013 bis 0,038 mm Schubspannungen(über 600 N/m²) auftreten, die mit großer Wahrscheinlichkeit Blut schädigend sind.

Aus US 5,158,440 ist eine Kreiselpumpe mit integriertem Motor bekannt geworden, die Mittel zum axialen hydrodynamischen Ausgleich für einen berührungslosen gehäusegelagerten Rotor aufweist. Diese Mittel beinhalten einen Ring, der im Spalt zwischen Rotor und Stator vorgesehen ist, um den Blutfluss zu reduzieren. In weiteren Ausführungsformen sind dabei am Gehäuse Einlaufkranzanordnungen vorgesehen, die den Blutfluss in diesem Spalt auf eine kleine Menge reduzieren.

Weiterhin ist aus US 5,470,208 eine Fluidpumpe mit magnetisch aufgehängtem Laufrad bekannt, wobei eine Pumpenkammer eine Einlassöffnung und eine Auslaßöffnung aufweist. In der Pumpenkammer ist ein drehbares Laufrad angeordnet. Dem Laufrad und dem Gehäusen sind polarisierte Elektromagneteinrichtungen zugeordnet, die den Antrieb des Laufrades bewirken. Das Laufrad wird dabei durch anhebende magnetische Kräfte stabilisiert, wobei eine erste Magnetkraft-Erzeugeeinrichtung Diamagnete enthält.

Eine kritische Würdigung des geschilderten Standes der Technik ergibt folglich, dass die berührungsfreie Rotation des Rotors einer Zentrifugalpume im Gehäuse entweder durch einen hohen Aufwand von Sensorik und elektromagnetischer Regelung oder auf Kosten einer hohen hydrodynamischen Belastung des Blutes durch schädigende Schubspannungen erreicht wird.

Der vorliegenden Erfindung liegt die Aufgabe zugrunde, eine Zentrifugal-Pumpe mit lagerlosem Rotor zu schaffen, bei der der Rotor auf einfache und flüssigkeitsschonende Weise in axialer und radialer Richtung stabilisierbar ist und die einen hohen Wirkungsgrad aufweist.

Die Aufgabe wird mit einer Zentrifugal-Pumpe mit den Merkmalen des Anspruchs 1 gelöst. Die Unteransprüche geben vorteilhafte Weiterbildungen and.

Die axiale Stabilisierung des Pumpenrotors erfolgt hydrodynamisch durch die durch den Rotor geförderte Flüssigkeit. Dazu kann der aufgrund des herrschenden Druckunterschieds zwischen dem radial äußeren Bereich und dem radial inneren Bereich des Pumpengehäuses auftretende, radial einwärts gerichtete Flüssigkeitsstrom zwischen den Rotoroberflächen und dem Gehäuse die hydrodynamischen Kräfte zur axialen Stabilisierung des Rotors erzeugen. Der Rotor ist symmetrisch zu seiner Mittelebene ausgebildet und weist eine obere und untere Abdeckung auf. Dadurch ergeben sich definierte Strömungsverhältnisse für den von der Peripherie einwärts gerichteten Verlustflüssigkeitsstrom, der zur axialen Stabilisierung des Rotors genützt werden kann.

Erfindungsgemäß wird also der strömungsbedingte Druckabfall im Seitenraum des axialsymmetrischen, beidseits gedeckten Pumpenrotors ausgenutzt: Der von der Pumpe generierte Ausgangsdruck am Rotorumfang wird über die Rückströmung durch die Rotorseitenräume zum Zentrum hin auf den Eingangsdruck der Pumpe reduziert. Dadurch, dass der Rotor und/oder das Pumpengehäuse derart geformt sind, dass die axialen Abstände zwischen der oberen und unteren Abdeckung des Rotors und der oberen und unteren Gehäusewandung im radial inneren Bereich des Rotors geringer sind als im radial äußeren Bereich, entsteht im zentralen Bereich der Abdeckungen eine Drosselwirkung beim Strömen der Flüssigkeit von außen nach innen. Der Drosselspalt bewirkt den größten Teil des Druckverlustes, wodurch im peripheren Bereich der Abdeckungen ein höherer Druck erhalten bleibt und somit eine Kraftkomponente auf die ganze Abdeckung ausgeübt wird, die einer Spaltverengung entgegenwirkt. Diese Kraft ist umso größer, je enger der Spalt im zentralen Bereich der Abdeckung wird.

Bewegt sich also der Rotor nach oben, so verengt sich der obere Spalt. Die dadurch bewirkte Krafterhöhung der Flüssigkeit drückt den Rotor dann wieder nach unten. Umgekehrt drückt die Flüssigkeit den Rotor wieder nach oben, sollte sich dieser aus der Mitte nach unten bewegen. So kommt es automatisch zu einer hydrodynamischen Stabilisierung der axialen Lage des Pumpenrotors. Der Drosselspalt wird bei axialer Auslenkung des Rotors in Richtung der Auslenkung verengt und auf der Gegenseite erweitert. Dies bewirkt auf der Seite des jeweils weiteren Spaltes eine relative Zunahme der radial einwärts gerichteten Rückströmung und damit einen asymmetrischen hydrodynamischen Druckabfall. Die Rotorseitenräume bilden dabei parallel-geschaltete Düsen, deren Differenzdruck auf der gesamten Oberfläche beider geschlossener Abdeckungen des Rotors wirksam ist und damit eine axial stabilisierende, symmetrisch zur Ruhelage des Rotors wirkende Rückstellkraft erzeugt. Der Rotor ist daher gegen Auslenkung in der z- Achse stabilisiert.

Gleiches gilt für das Verkippen des Rotors, also Rotationsbewegung um die x- und y-Achsen. Auch hier werden die jeweils achsensymmetrischen Drosselspalten gegensinnig verengt und erweitert mit der Folge einer Rückstellkraft in Richtung einer stabilen Rotorposition im Gehäuse. Diese Effekte sind bisher in lagerlosen Pumpen nicht genutzt worden. Eine wesentliche Voraussetzung für die wünschenswerte Minimierung der erforderlichen Stabilisierungsenergie ist die erfindungsgemäße Axialsymmetrie des Pumpenrotors und der Rotorseitenräume.

Die Abstände zwischen der oberen und unteren Rotorabdeckung und den Gehäuseober- und -unterseiten verringern sich dabei kontinuierlich.

Die radiale Stabilisierung des Rotors kann rein passiv aufgrund von Reluktanzkräften erfolgen. Dabei sollte der hydrodynamische Radialschub, der den Rotor aus dem Zentrum der Drehbewegung drängt, minimiert werden. Dazu wird vorzugsweise die Projektionsfläche des Rotors in der x-y/z-Ebene als Wirkfläche radial destabilisierender Druckkräfte minimal gehalten. Sie wird lediglich durch die Dicke der Abdeckungen des Rotors bestimmt. Der Rotor kann vorzugsweise vollständig aus para- und/oder ferromagnetischem Material gefertigt und permanent magnetisiert sein. Es ist dann keine Verkapselung diskreter Permanentmagnete erforderlich, die zur Vergrößerung der radialen Projektionsfläche beiträgt. Eine weitere Maßnahme gegen radiale Instabilität besteht darin, einen zirkulären rotationssymmetrischen Ringspalt vorzusehen, der die Rotorseitenräume von einem tangential den Förderstrom abführenden Spiralkanal trennt. Hierdurch wird ein konstanter Strömungswiderstand gegenüber dem Fluid, das aus dem Rotor austritt, erzeugt und damit eine über den Umfang des Rotors gleichförmig wirkende radiale Druckkraft auf den Rotor. Die dritte Maßnahme gegen radiale Instabilität besteht in einer geeigneten Gestaltung des strömungsabführenden Spiralkanals, bei der radial destabilisierende Druckkräfte im Bereich der Zunge vermieden werden.

Der Antrieb der Pumpe erfolgt vorzugsweise als permanenterregte elektrische Synchronmaschine, deren Rotor vom Rotor der Pumpe gebildet wird, welcher sich zwischen zwei symmetrischen Statoren mit großem Luftspalt befindet. Die Statoren können gegeneinander verdreht sein, ebenso die in zwei Ebenen geteilten permanentmagnetischen Bereiche des Rotors. Die Polüberdeckungen und die Topologie der elektromagnetischen Durchflutung können dahingehend optimiert werden, dass bei geringster Masse an Magnetmaterial ein hoher Wirkungsgrad bei geringer Welligkeit, d. h. hohe Konstanz des Drehmoments und niedriger axialer Steifigkeit des Magnetsystems besteht, wodurch die hydrodynamische Levitation des Rotors ermöglicht wird. Gleichzeitig werden die magnetischen Reluktanzkräfte zwischen Statoren und Rotor zu dessen radialer Zentrierung herangezogen.

Die erfindungsgemäße Zentrifugal-Pumpe eignet sich insbesondere für den Einsatz als Blutpumpe und dabei zur Implantation in den menschlichen Körper, da sie nur wenig Platz benötigt und absolut wartungsfrei ist. Die Pumpe kann als Blutpumpe zur Unterstützung der Herztätigkeit eines Patienten oder im Zusammenhang mit einer Herz-Lungen-Maschine eingesetzt werden. Des Weiteren kann die Pumpe zur Förderung anderer Flüssigkeiten, insbesondere von aggressiven und gefährlichen Flüssigkeiten oder empfindlichen Flüssigkeiten, bei denen ein Kontakt zur Außenwelt vermieden werden sollte, eingesetzt werden. Die Komponenten der Pumpe, die mit der Flüssigkeit in Kontakt kommen, können mit einer auf die Flüssigkeit abgestimmten Beschichtung versehen sein.

Nachfolgend werden bevorzugte Ausführungsbeispiele der erfindungsgemäßen Zentrifugalpumpen, insbesondere für den Einsatz als permanent implantierbare Blutpumpen zur Herzunterstützung, anhand von Zeichnungen näher beschrieben und in ihrer Funktion erläutert.

Es zeigen:
- Fig. 1: einen Querschnitt durch ein Ausführungsbeispiel einer erfindungsgemäßen Zentrifugalpumpe;
- Fig. 2a: eine perspektivische Ansicht des Rotors der Pumpe aus Fig. 1 mit teilweise entfernter oberer Abdeckung;
- Fig. 2b: einen schematischen Querschnitt durch Rotor und Gehäuse der Pumpe aus Fig.1 zur Verdeutlichung der geometrischen Kenngrößen;
- Fig. 3a, 3b: eine schematische Darstellung der Strömungsverhältnisse durch den Rotor einer Zentrifugalpumpe mit konstanter und mit radial einwärts abnehmender Weite des Rotorseitenraums;
- Fig. 4: Diagramme mit dem radialen Verlauf der Drücke in den Rotorseitenräumen
a) bei axialer mittiger Position des Rotors im Gehäuse,
b) bei axialem Versatz des Rotors zum Einlass des
Gehäuses hin;
- Fig. 5: Diagramm über den Verlauf der Rückstellkraft in Abhängigkeit von der axialen Auslenkung aus der mittigen Rotorposition für zwei unterschiedlich weite Drosselspalte;
- Fig. 6: Diagramme mit dem radialen Verlauf der Drücke in den Rotorseitenräumen bei Verkippen des Rotors im Gehäuse;
- Fig. 7a, b, c: Querschnitte durch verschiedene Ausführungsformen des Drosselspaltes bei erfindungsgemäßen Zentrifugalpumpen;
- Fig. 8: Drosselkurven der erfindungsgemäßen Pumpe bei unterschiedlichen Drehzahlen;
- Fig. 9: Diagramme mit den hydraulischen Wirkungsgraden über dem Volumenstrom der Pumpe aus Fig. 1 bei unterschiedlichen Drehzahlen.

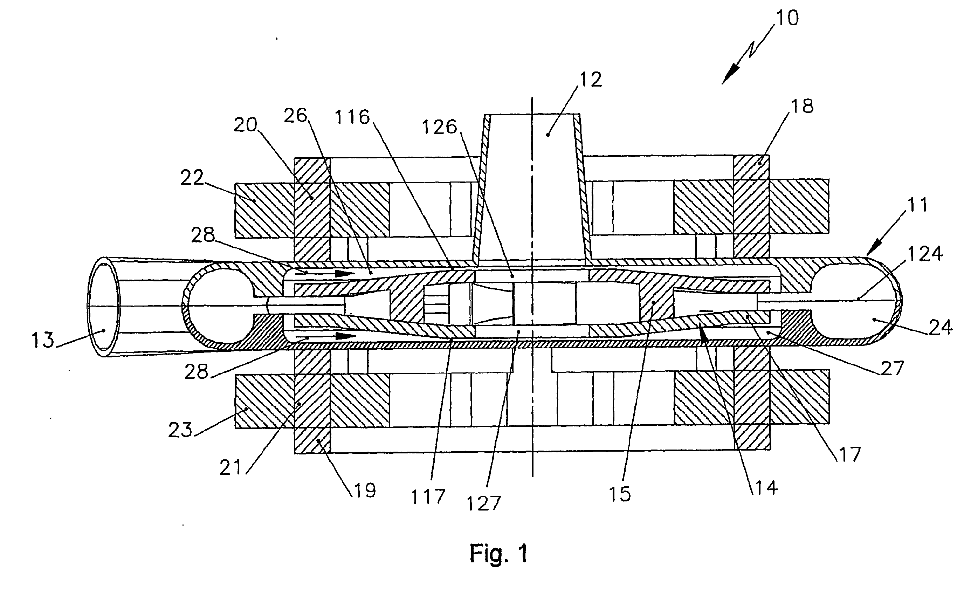

Die Zentrifugal-Pumpe 10 aus Fig. 1 weist ein Pumpengehäuse 11 mit einem Einlass 12 und einem tangentialen Auslass 13 für Blut oder eine andere Flüssigkeit auf. Ansonsten ist das Pumpengehäuse 11 flüssigkeits- und gasdicht verschlossen. Im Inneren des Pumpengehäuses 11 ist ein Pumpenrotor 14 lagerlos und drehbar angeordnet. Mit Hilfe des Pumpenrotors 14 wird das durch die Öffnung 12 einströmende Blut radial nach außen und zum Auslass 13 hin befördert. Dazu weist der Pumpenrotor Förderschaufeln 15 auf. Die Förderschaufeln 15 sind durch eine obere Abdeckung 16 und eine untere Abdeckung 17 nach oben und unten abgedeckt.

Der Pumpenrotor 14 weist mindestens in seinem Umfangsbereich permanent magnetisierte Bereiche auf. Im dargestellten Beispiel ist er jedoch vollständig aus einem para- und/oder ferromagnetischen Material gefertigt. Er bildet damit gleichzeitig den Rotor für einen Antriebsmotor, der neben dem Rotor 14 zwei ringförmige Statoren 18, 19 aufweist, die eine Vielzahl gleichmäßig verteilter, in Richtung des Rotors 14 vorstehender Abschnitte 20, 21 aufweisen, die in einer bevorzugten Ausführungsform jeweils von einer Wicklung 22, 23 umgeben sind. Es sind jedoch auch Wicklungsanordnungen denkbar, bei denen mehrere der axial vorstehenden Abschnitte 20, 21 von einer Wicklung 22, 23 umfasst werden. Die beiden Statoren 18, 19 sind außerhalb des Gehäuses angeordnet, sodass der Antrieb des Pumpenrotors 14 berührungslos erfolgt. Dadurch entstehen nur minimale Belastungen für das zu fördernde Blut im Gehäuse 11. Der Rotor 14 und das Gehäuse 11 sind vorzugsweise dort, wo sie mit Blut in Kontakt kommen, mit einem blutverträglichen Material überzogen.

Die Statoren 18, 19 erzeugen einen rotierenden Magnetfluss im Zwischenraum zwischen den Bereichen 20, 21 und dem Rotor 14. Gleichzeitig sorgen die beiden Statoren für eine radiale Zentrierung des Rotors 14 im Gehäuse 11. Die radiale Zentrierung erfolgt dabei rein passiv aufgrund von Reluktanzkräften.

Es wird im Folgenden gezeigt, auf welche Weise bei der Pumpe 10 die Stabilisierung des Pumpenrotors 14 gegen Verschiebung im Gehäuse 11 in axialer Richtung durch hydrodynamisch generierte Druckkräfte erfolgt.

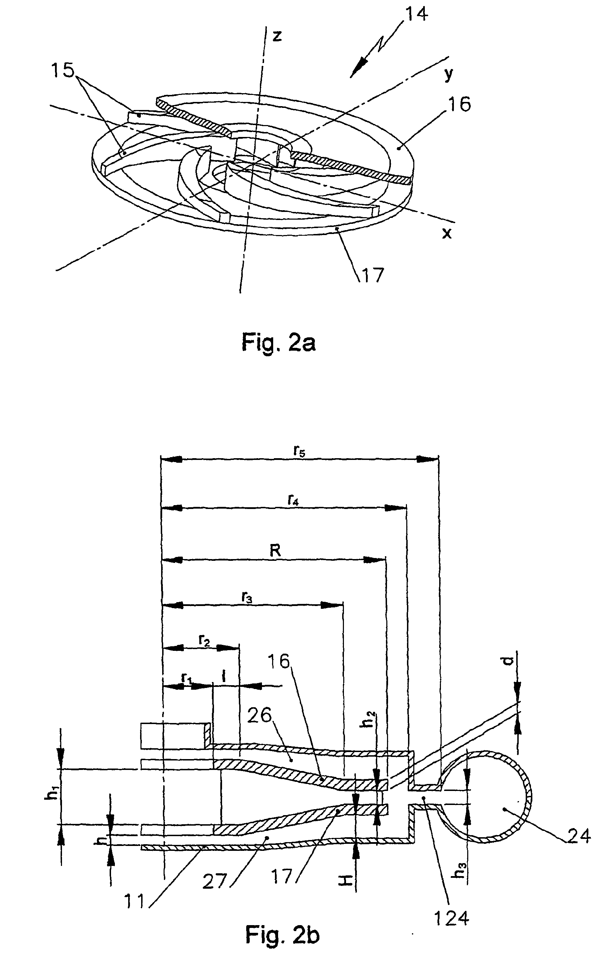

Der Rotor 14 weist Abdeckungen 16, 17 auf, die vollständig axialsymmetrisch und glatt sind und Schaufeln 15 umschließen, deren Zahl und Form nach strömungsdynamischen Erfordernissen optimiert werden kann (Fig. 2a). In den hier dargestellten Ausführungsformen beträgt die Schaufelzahl sechs. Das Blut tritt saugseitig durch eine Öffnung 126 in den Rotor 14 ein. Eine zweite gleichgroße Öffnung 127 in der unteren Abdeckung dient zum Druckausgleich zwischen Rotorseitenräumen 26, 27, sodass unabhängig von den dort herrschenden Druck- und Strömungsverhältnissen an den dem Zentrum zugewandten Rändern der Öffnungen 126, 127 (Fig. 1) stets der gleiche Druck herrscht. Das Blut verlässt den Rotor 14 nach Passage der Schaufelkanäle am äußeren Umfang in den abführenden Ringspalt 124 und schließlich in den Spiralkanal 24 unter hohem Druck. deckungen 16, 17 des Rotors 14 und den Wänden des Gehäuses 11 begrenzt. In einer Zentrifugalpumpe mit gedecktem Pumpenrotor 14 herrschen in diesen Rotorseitenräumen 26, 27 zwei Strömungsrichtungen: Die unmittelbar an den rotierenden Abdeckungen 16, 17 anhaftende Grenzschicht wird durch die Zentrifugalkraft nach außen bewegt, während der aufgebaute Druck nahe dem stationären Gehäuse 11 und im überwiegenden Volumen des Seitenraumes 26, 27 eine radial nach innen gerichtete Rückströmung 28 erzeugt, die als Kurzschluss- oder Verlustströmung bezeichnet wird, die den hydraulischen Wirkungsgrad vermindert. Im technischen Pumpenbau dienen u. a. achsnahe Labyrinthdichtungen zur Verminderung dieser Verluste. Für Blutpumpen verbietet sich ihre Anwendung wegen der Ausbildung hoher Schubspannungen und schlecht durchspülter Totwasser.

Die im Weiteren zur Beschreibung der Geometrie von Pumpenrotor und Gehäuse verwendeten Bezeichnungen sind in Fig. 2b erläutert. Es bedeuten:
- R: Radius der Abdeckung des Rotors;
- r₁: Radius der Zuströmöffnungen in den Rotor;
- r₂: Radius am Eintritt in den Drosselspalt;
- r₃: Radius am Beginn der permanentmagnetischen Bereiche der Abdeckungen;
- r₄: Innenradius des Gehäuses;
- r₅: Außenradius des zirkulären Ringspaltes;
- H: Weite des Rotorseitenraums am Rotorumfang;
- h: Axiale Weite des Drosselspaltes;
- h₁: Schaufelhöhe am Rotoreintritt;
- h₂: Schaufelhöhe am Rotorumfang;
- h₃: Höhe des zirkulären Ringspaltes;
- I: radiale Länge des Drosselspaltes (r₂ - r₁);
- d: Dicke der Abdeckungen.

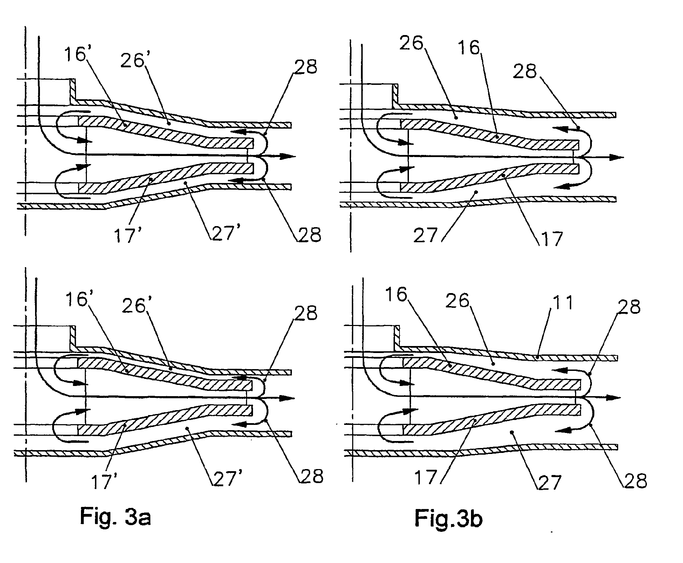

Bei den erfindungsgemäßen Pumpen wird der unvermeidliche Energieverlust der radialen Rückströmung 28 im Rotorseitenraum 26, 27 zur Erzeugung einer hydrodynamischen Rückstellkraft gegen axiales Auswandern des Rotors durch eine neuartige geometrische Gestaltung dieses Spaltraumes 26, 27 genutzt, wie in Fig. 3 verdeutlicht ist.

In Fig. 3a ist ein Rotorseitenraum mit über den Radius der Abdeckung 16', 17' in konstanter axialer Weite dargestellt. In der Mittellage des Rotors ergeben sich gleiche Stromstärken in beiden Rotorseitenräumen, weil am Umfang und im Zentrum des Rotors die Drücke gleich sind und damit symmetrische Druckverteilungen auf die Abdeckungen wirken. Weicht der Rotor in Fig. 3 nach einer Seite axial aus, wird im engeren Rotorseitenraum die Strömung beschleunigt und folglich nimmt der Druck auf die Abdeckung dort ab, und umgekehrt im erweiterten gegenüberliegenden Rotorseitenraum bei hier verzögerter Strömung zu, folgend dem Gesetz von Bernoulli und in Analogie zu dem bekannten "hydrodynamischen Paradoxon". Die Ausweichbewegung verstärkt sich daher bis zum Anlaufen des Rotors an der Wand des Gehäuses.

Die erfindungsgemäße geometrische Gestaltung der Rotorseitenräume 26, 27 kehrt diesen Effekt um (Fig. 3b): Die Spalte zwischen Abdeckungen 16, 17 und Gehäuse 11 weisen an ihren radial inneren Bereichen jeweils eine - relativ zum restlichen Spalt - starke axiale Verengung von kurzer radialer Erstreckung auf, sodass unmittelbar vor der Eintrittsöffnung des Rotors beidseitig ein symmetrischer Drosselspalt 116, 117 (Fig. 1) ausgebildet ist. Die Verlustströmung 28 trifft hier auf einen hohen terminalen seriellen Widerstand. Bei mittiger Position des Rotors herrscht Druckgleichheit im oberen und unteren Rotorseitenraum. Bei axialer Auslenkung des Rotors wird der Anteil des Drosselspaltes im enger werdenden Spalt am gesamten Spaltwiderstand immer größer. Dadurch bleibt der Förderdruck am Rotorumfang im verengten Rotorseitenraum radial einwärts bis nahe an den Drosselspalt annähernd erhalten und wird erst dort über den terminalen Widerstand der Drossel steil abgebaut. Im größer werdenden gegenüberliegenden Spalt wird der Einfluss der Drosselstelle immer kleiner. Der Druck wird radial einwärts, beginnend am Umfang, gleichmäßig über die gesamte Spaltlänge abgebaut.

Die resultierende Differenz der Druckkräfte auf die geschlossenen Abdeckungen bewirkt daher bei jeder axialen Auslenkung des Rotors aus der Mittellage eine der Abweichung proportionale Kraft, die den Rotor zurückstellt. Es versteht sich, dass in dem zum Gehäuse axial planparallelen ringförmigen engen Drosselspalt 116 selbst das oben genannte Strömungsgesetz weiter gilt, wonach die beschleunigte Strömung im engeren Spalt den Rotor in Richtung der Verengung destabilisieren würde. Die Wirkfläche der Druckkräfte ist hier jedoch nur ein geringer Bruchteil der Oberfläche der Abdeckungen, sodass die axial rückstellenden Kräfte weit überwiegen.

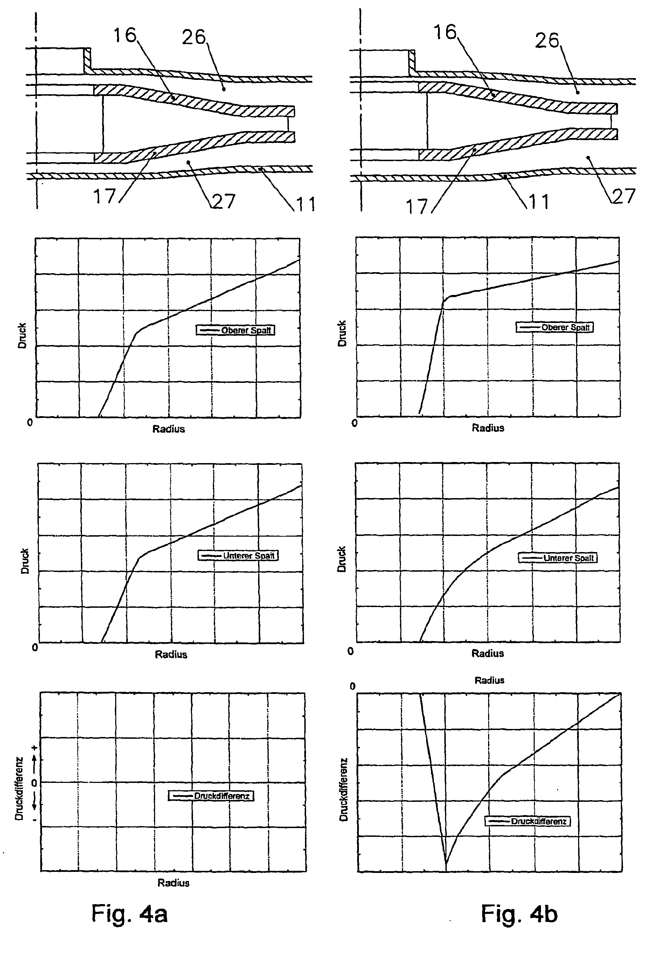

Messungen an einem Rotor (R = 20 mm) bei einer Pumpe nach Fig. 1 haben für einen axialen Versatz des Rotors radiale Druckverläufe in den Rotorseitenräumen ergeben, die in Fig. 4a, b dargestellt sind. Jeder axiale Versatz (Fig. 4b) bewirkt eine Asymmetrie des radialen Verlaufes der Druckabnahme im Rotorseitenraum. Die resultierende Druckdifferenz wird auf der weit überwiegenden Fläche der Abdeckungen als rückstellende Kraft wirksam, die den Rotor axial mittig positioniert.

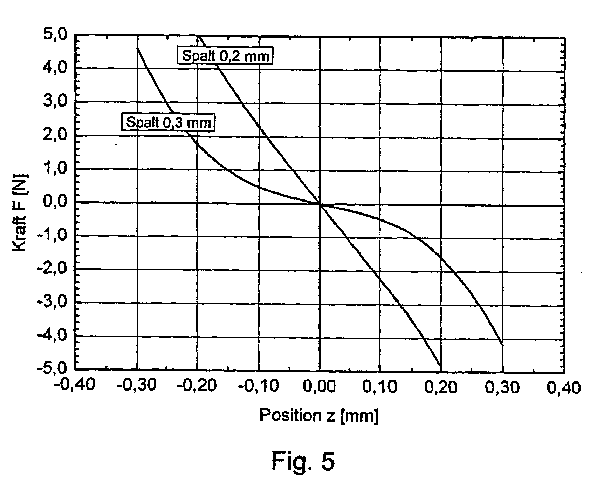

Die Beträge der Rückstellkräfte, die aus den Druckdifferenzen aus Fig. 4 resultieren, sind in Fig. 5 dargestellt. Erwünscht ist eine möglichst hohe Steifigkeit dF/dz über den gesamten Bereich der Auslenkung in Richtung z. (Fig. 2a) Man erkennt, dass ein enger Drosselspalt (h = 0,2 mm) diese Forderung linear und steiler erfüllt als ein weiterer Spalt (h = 0,3 mm), wobei in beiden Fällen bei maximaler Auslenkung Rückstellkräfte von ca. ± 5 N erreicht werden, dies weitgehend unabhängig von Arbeitsdruck und Förderstrom der Pumpe (120 ± 20 mmHg, 5 ± 2 I/min). Für die Geometrie des Drosselspaltes hat sich ein Verhältnis h/R im Bereich 0,016 bis 0,008 und von I/R im Bereich von 0,16 bis 0,08 als besonders günstig erwiesen, entsprechend Höhen des Drosselspaltes von 0,32 mm bis 0,16 mm und einer axialen Länge von 1,6 bis 3,2 mm in der bevorzugten Ausführungsform mit einem Radius R des Rotors von 20 mm. Im Bereich h/R < 0,006 nimmt bei wachsenden Scherkräften die Steifigkeit nicht weiter zu. Im Bereich h/R > 0,2 werden keine ausreichenden Rückstellkräfte erreicht.

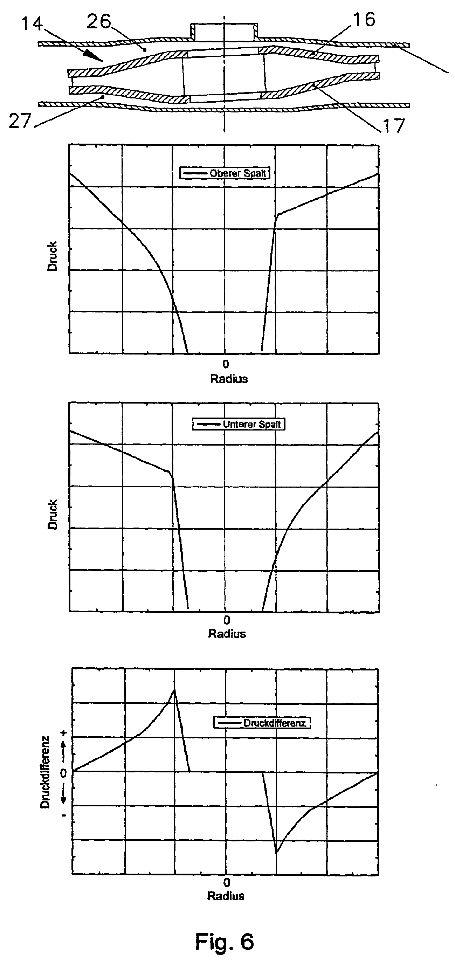

Eine rotatorische Auslenkung des Rotors (Fig. 6) um die Achsen (x, y), also ein Verkippen im Gehäuse, bewirkt symmetrisch zur Rotationsachse eine gegensinnige Verengung und Erweiterung der Drosselspalte und damit unterschiedliche radiale Verläufe der Verlustströmungen und der Druckabnahme in gegenüberliegenden Rotorseitenräumen. Der resultierende Differenzdruck wird als rückstellende Druckkraft auf der gesamten Fläche beider Abdeckungen wirksam und dreht den Rotor in seine neutrale Position zurück.

Bei einer typischen Geometrie des Drosselspaltes von (h/R) = 0,01 und einer radialen Lage des Eintritts in den Drosselspalt von (r₂/R) = 0,35 kann ein Rotor von R = 20 mm um einen Winkel von 1,6° verkippen, wenn der Drosselspalt radial verschlossen ist und der Rotor tangential punktförmig am Radius r₂ anläuft. Die Spaltweite H am Rotorumfang ist in der bevorzugten Ausführung im Bereich von H/R = 0,05 ± 0,01 als günstig erkannt worden (H = 0,8 - 1,2 mm). Die gewählte Geometrie schließt ein Anlaufen des Rotors in Bereichen höherer Umfangsgeschwindigkeiten am Radius R aus, so dass der Eintritt der stabilisierenden Rückströmung in den Rotorseitenraum nicht behindert wird.

Die erfindungsgemäße Gestaltung der Rotorseitenräume und der Drosselspalte bewirken somit eine räumliche Stabilisierung des Rotor in seine geometrisch neutrale, symmetrische Position im Gehäuse gegen Translation in Richtung ± z und Rotation um die Achsen x und y.

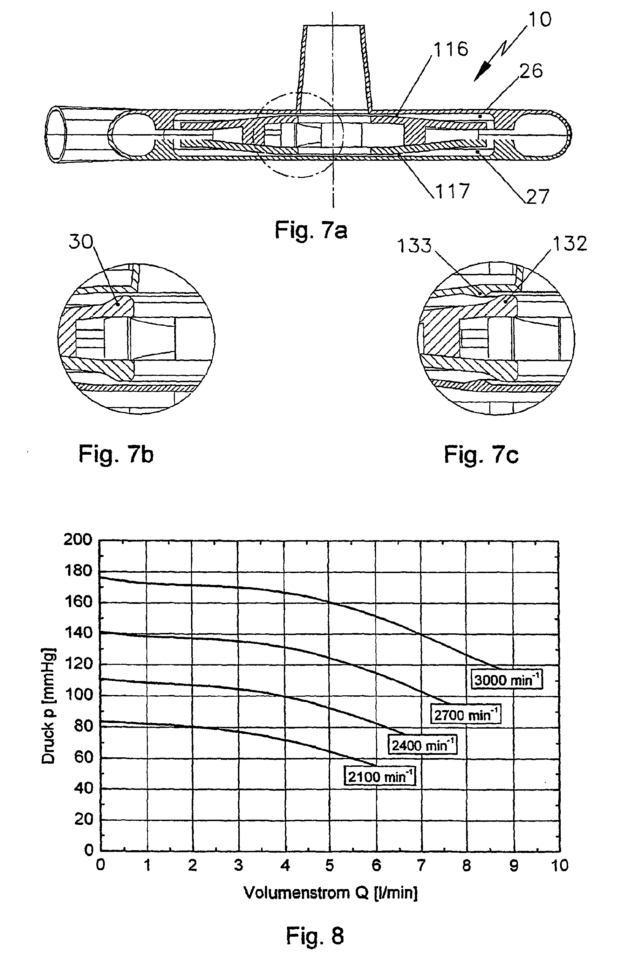

Weitere mögliche Ausführungen von Drosselspalten bei erfindungsgemäßen Pumpen sind in Fig. 7 dargestellt. Bei der Pumpe nach Fig. 1 verjüngen sich die Rotorseitenräume 26, 27 stetig vom Radius R bis zum Eintritt in den Drosselspalt 116, 117 am Radius r₂, wobei der Spalt bis zum Einlass in den Rotor am Radius r₁ eine konstante Höhe h aufweist, wie Fig. 7a zeigt. In Fig. 7b ist eine mögliche Variante dargestellt, bei welcher der Drosselspalt unter Beibehaltung der Verhältnisse h/R (s. o.) durch einen Wulst 30 gebildet wird, der aus den Abdeckungen 16, 17 im Bereich der Radien r₂ bis r₁ ausgeformt ist. Auf diese Weise wird der überwiegende Teil der Rotorseitenräume weiter gehalten, sodass dort die viskose Reibung zwischen rotierendem Rotor und stationärem Gehäuse vermindert wird, wobei die stabilisierende Wirkung des Spaltes erhalten bleibt. In Fig. 7c ist eine mögliche Variante gezeigt, bei der die Form der Abdeckungen mit ausgebildetem Wulst 132 der Fig. 7b entspricht. Zusätzlich ist hier am Gehäuse ein radial weiter außen gelegener Wulst 133 ausgeformt, wobei die Spalthöhe weiter die erfindungsgemäßen Verhältnisse h/R beibehält. In dieser Ausführung wird neben der axial stabilisierenden Wirkung der Drossel eine radiale Kraftkomponente erzeugt, welche die Zentrierung des Rotors unterstützt.

Die rückstellenden Druckkräfte werden bei jeglicher Lageabweichung und damit Änderung der Strömungsgeometrie sehr schnell wirksam, nämlich mit der Ausbreitungsgeschwindigkeit der hervorgerufenen Druckänderung im inkompressiblen Fluid, also der Schallgeschwindigkeit. Diese beträgt im Blut (ähnlich Wasser) ca. 1500 m/s (Luft: ca. 300 m/s). Für die dargestellte Geometrie der bevorzugten Ausführung errechnet sich damit eine Verzögerung der Wirksamkeit einer Lageabweichung des Rotors von ca. 50 Mikrosekunden.

Instationäre numerische Simulationsrechnungen unter Berücksichtigung von Beschleunigungs- und Trägheitskräften haben ergeben, dass sprungförmige Lageänderungen des Rotors innerhalb einer bis zweier Umderhungen vollständig ausgeregelt werden. Bei sinusförmiger axialer Relativbewegung des Rotors gegenüber dem Gehäuse ergibt sich eine Phasenverschiebung zwischen Auslenkung und Rückstellung von ca. 10 ms, entsprechend etwa einer halben Rotorumdrehung.

Ein derart schnelles Ansprechen der Lageregelung ist insbesondere beim Anlauf der Pumpe von Vorteil. Im Stillstand befindet sich der Rotor in einer zufälligen, axial verschobenen oder gekippten Position im Gehäuse. Die stabilisierenden Druckkräfte werden erst im Betrieb generiert. Eine hydrodynamische Levitation des Rotors innerhalb seiner ersten wenigen Umdrehungen verhindert eine reibungsbedingte Strukturschädigung von Rotor und Gehäuse.

Die Schnelligkeit und Steifigkeit der erfindungsgemäßen Lageregelung des Rotors ist auch dann von Vorteil, wenn der Träger einer implantierten Blutpumpe im Alltagsleben wechselnden Beschleunigungen in unterschiedlichen Raumachsen ausgesetzt ist. Die demonstrierten Ausregelungszeiten von ca. 10 ms (100 Hz) mit der Steifigkeit von ca. 20 N/mm lassen erwarten, dass auch bei mehrfacher Erdbeschleunigung ein Anstoßen des Pumpenrotors am Gehäuse sicher vermieden wird.

Radiales Auswandern des Rotors, also Translation in den Richtungen ± x und ± y, wird von der oben beschriebenen axialen Lagestabilisierung durch Druckkräfte auf die Abdeckung nicht verhindert. Die radiale Stabilisierung des Rotors geschieht passiv durch Reluktanzkräfte. Dabei ist es von Vorteil, den in jeder Zentrifugalpumpe auftretenden destabilisierenden Radialschub gering zu halten und zu kompensieren. Dies gelingt durch mehrere Maßnahmen:

Der Radialschub nimmt proportional mit der Gesamthöhe (h₂ + 2d) des Rotors am Umfang zu. Dieser wird daher besonders flach gehalten, wobei sich Verhältnisse von (h₂/R) im Bereich von 0,08 bis 0,12 und von (d/R) im Bereich 0,05 bis 0,1 als günstig erwiesen haben. Eine besonders flache Bauweise wird durch die erfindungsgemäße Herstellung der Abdeckungen aus vollem, biokompatibel beschichtetem Magnetmaterial ermöglicht, wodurch eine Verkapselung diskreter Magnete entfallen kann.

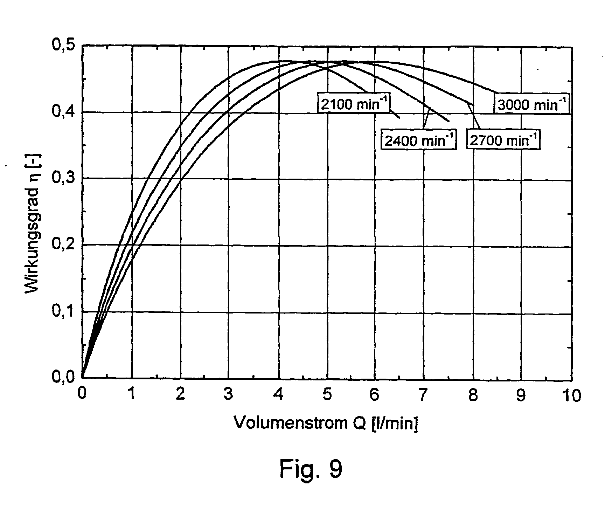

Der Radialschub (SR) nimmt exponentiell zu, wenn der Rotor nicht im Bestpunkt (Qₒₚₜ) seines Wirkungsgrades arbeitet: Sir ∼ 1 - (Q/Qₒₚₜ)² (Bohl, W., Strömungsmaschinen 2, 8. Auflage, Vogel Fachbuchverlag, Würzburg (2002)). Aus Fig. 9 ist ersichtlich, dass die optimalen Wirkungsgrade bei den erwarteten Drehzahlen von 2400 - 3000 min⁻¹ im Arbeitsfeld der Pumpe von 5 ± 2 \Imin erreicht werden, wodurch der Radialschub weiter minimiert wird.

Die Abführung des Volumenstromes durch einen Spiralkanal mit tangentialem Auslass führt dann zu einem Radialschub, wenn im Bereich der Zunge (Schneide) im Arbeitsbereich ein Drucksprung auftritt. Erfindungsgemäß wird dies weitgehend verhindert, in dem das vom Rotor geförderte Volumen vor dem Eintritt in den Spiralkanal einen zirkulären Ringspalt passiert, wobei eine Gleichrichtung der Strömung eintritt. Der weitere Abstrom des Fluids erfolgt danach über den Spiralkanal, der als archimedische Spirale mit annähernd kreisförmigem Querschnitt ausgeführt sein kann. Als günstig im Sinne der Erfindung haben sich dabei erwiesen eine axiale Höhe h₃ des Ringspaltes von h₃/h₂ im Bereich von 0.6 bis 2.0, eine radiale Länge (r_{4/}r₅) im Bereich von 0.8 bis 1.0, sowie eine über den Umfang des Spiralkanals stetig zunehmender Kreisquerschnitt mit Durchmesser h₃ an der Zunge und einem Enddurchmesser an der tangentialen Abströmung von 4. h₃. In dieser bevorzugten Geometrie tritt im Bereich der Maxima des Wirkungsgrades kein radial destabilisierender Drucksprung im Bereich der Zunge auf.

Die drei beschriebenen Merkmale zur Verminderung des Radialschubs können diesen weitgehend vermindern, nicht jedoch kompensieren.

Hierzu werden die bereits erwähnten, durch die Stator- und Rotorgeometrien erzeugten magnetischen Reluktanzkräfte herangezogen.

Der hydraulische Wirkungsgrad einer implantierbaren Blutpumpe soll möglichst hoch sein, da jegliche Verlustleistung aufgrund von viskoser Reibung, Wirbelbildung und Kurzschlussströmungen zur Blutschädigung beiträgt und letztlich als Wärmeenergie auf das Blut übertragen wird. Hinzu kommt, dass die Leistungsanforderungen an den elektrischen Antrieb und den hierfür notwendigen Energieaufwand umgekehrt proportional abnehmen, was die geforderte Miniaturisierung begünstigt.

Durch die beschriebene Geometrie der Radseitenräume und der abführenden Kanäle, und in Verbindung mit einem gedeckten Rotor mit optimaler Beschaufelung, werden bisher unbekannte Wirkungsgrade erzielt. Dies ist in Fig. 8 und Fig. 9 anhand gemessener Drosselkurven und zugehöriger Wirkungsgrade demonstriert. Im typischen Arbeitsbereich der Pumpe von 5 ±2 I/min Volumenstrom gegen Drücke von 120 ± 20 mmHg betragen die hydraulischen Pumpleistungen (p · V) 1,4 ± 0,7 Watt. Die hierfür erforderlichen Drehzahlen liegen im Bereich von 2400 - 3000 min⁻¹.

Bei Verwendung eines Testfluides der Viskosität von Blut (4 mPas) weist die Pumpe aus Fig. 1 die in Fig. 9 gezeigten Wirkungsgrade auf, die im typischen Arbeitbereich 0,4 bis 0,47 betragen. Dies entspricht etwa dem Vierfachen der Werte, die für bekannte Blutpumpen angegeben wurden.

Entsprechend niedrig ist die erforderliche Wellenleistung von ca. 3 ± 1,5 Watt als besonders günstige Voraussetzung für die Miniaturisierung von Antrieb und Energieversorgung.

Zur erfindungsgemäßen hydrodynamischen Stabilisierung des Rotors werden die wirkungsgradmindernden Rückströmungen im Rotorseitenraum genutzt. Diese betragen im typischen Arbeitsbereich ca. 2 - 3 I/min. Um einen Netto-Volumenstrom von 5 l/min zu erzeugen, müssen demnach 7 - 8 I/min vom Rotor gefördert werden. Dies entspricht einer hydraulischen Mehrleistung von 0,5 bis 0,8 Watt für die Stabilisierung des Rotors und damit nur einem Mehrbedarf von etwa 20% der Wellenleistung. Dieser muss jedoch nicht zusätzlich aufgebracht werden, sondern entstammt der in anderen Pumpen ungenutzt verschwendeten/vernichteten Energie der Verlustströmungen.

Die erfindungsgemäße Pumpe ist besonders wirksam und soll dabei das Blut möglichst schonend fördern. Die wichtigste Voraussetzung dafür ist die Vermeidung hoher Schubspannungen. Die Blutpumpe unterscheidet sich von bekannten Pumpen mit hydrodynamischen Gleitlagern u. a. dadurch, dass die Spalträume zwischen Rotor und Pumpengehäuse weit gehalten sind. Selbst in dem kleinen Bereich der achsnah langsam drehenden Drosselspalte betragen die Spalthöhen im Vergleich zu bekannten Pumpen mit hydrodynamischer Lagerung durch Keilflächen ein Vielfaches der dort angegebenen Werte und entsprechend gering sind die auftretenden Schubspannungen.

Bei der maximal im Betrieb zu erwartenden Drehzahl des Rotors von 3000 min⁻¹ beträgt die Umfangsgeschwindigkeit im Drosselspalt lediglich Y = 1,8 m/s und bei einer Spalthöhe h von 0,2 mm errechnet sich ein Schergrad ã = γ/h von 9000 s⁻¹. Mit einer typischen Viskosität (η) des Blutes von 4 mPas ergibt sich daraus eine mittlere Schubspannung T = γ · η von 36 Nm⁻². Diese liegt damit um eine Größenordnung unterhalb des Grenzwertes von 400 Nm⁻², der nach neueren Untersuchungen als kritisch für Blutschädigung durch Scherkräfte angesehen wird (Paul, R., et al., Shear stress related blood damage in laminar couette flow. Artif Organs, 2003. 27(6): p. 517-29).

Die kumulative Traumatisierung (BT) eines Blutvolumens (V) bei Passage einer Zone hoher Schubspannung T korreliert auch mit der Expositionszeit (t) nach der Beziehung BT ∼ (V · T · t). Numerische Simulationsrechnungen haben ergeben, dass die mittlere Passagezeit des Blutes durch die gesamte Pumpe bei einem Volumenstrom von 5 I/min ca. 100 ms beträgt, wobei ein Teilchen im Drosselspalt höchstens für ca. 0,3 ms in einem Scherfeld von unter 40 N/m² verweilt. Das momentane Blutvolumen in den Drosselspalten beträgt lediglich 15 mm³. Derart günstige strömungsdynamische Voraussetzungen für eine niedrige Blutschädigung sind bisher nicht mitgeteilt worden.

Neben der mechanischen Zellzerstörung durch Schubspannungen und Wandkontakte ist die Aktivierung der zellulären und molekularen Mechanismen der Blutgerinnung ein zentrales Problem implantierbarer Blutpumpen. Gerinnsel, die Pumpen blockieren oder fortgeschwemmt als Embolien Komplikationen, z. B. Schlaganfälle und Niereninfarkte, hervorrufen können, entstehen bevorzugt durch Kontaktaktivierung an Fremdoberflächen und ebenso in Totwassern und stehenden Wirbeln. Die erfindungsgemäße Blutpumpe weist keine derartigen Strömungsbereiche auf, vielmehr sind alle Blut führenden Räume ständig mit hoher Strömungsgeschwindigkeit und kurzer Verweilzeit gespült. Die Oberfläche aller stationären und rotierenden Bauteile ist glatt und in Strömungsrichtung sind Stufen und Profilierungen vermieden. Alle bevorzugten Ausführungen können aus biokompatiblen Werkstoffen hergestellt und mit einer gerinnungshemmenden Beschichtung, z. B. durch oberflächengebundenes Heparin, versehen sein.

Aus den bisher dargestellten erfindungsgemäßen Mechanismen zur hydrodynamischen Levitation des Pumpenrotors folgt schließlich, dass der Rotor in axialer Richtung durch die Effekte des Drosselspaltes mittig im Gehäuse positioniert wird. In radialer Richtung bedarf es der zusätzlichen Unterstützung magnetischer Reluktanzkräfte. Im Betrieb wird sich der Rotor dynamisch verschieben, taumeln und exzentrisch laufen. Aufgrund des ausreichenden Abstandes zu den Wandungen und der schnellen Reaktionszeit der stabilisierenden Kräfte wird er aber zu keiner Zeit anlaufen. Die gewisse Beweglichkeit des Rotors im Gehäuse ist ausdrücklich erwünscht, da nur in dieser erfinderischen Kombination der genannten Merkmale ein sicherer Betrieb, ein hoher Wirkungsgrad, ständige Spülung aller Blut führenden Räume und geringste Blutschädigung erreicht werden können.

Der berührungslose Antrieb des Pumpenrotors kann in einer bevorzugten Ausführung in Form einer permanentmagnetisch erregten zweisträngigen elektrischen Scheibenläufer-Synchronmaschine erfolgen.

Die hydrodynamische Levitation des Rotors der Pumpe bedingt für den elektrischen Antrieb einen nachteilig großen magnetischen Luftspalt zwischen den Statorköpfen. Dieser ist erforderlich für die Aufnahme aller Gehäusewandungen, der blutführenden Rotorseitenräume und des Rotors selbst. Der Luftspalt muss L = 0,1 bis 0,15. R betragen und stellt damit im Motorenbau unübliche Anforderungen an die Optimierung der elektromechanischen Effizienz des Antriebs.

Ein zu erfüllendes Kriterium ist es, mit einem Minimum an Masse der Permanentmagnete und damit geringer axialer magnetischer Steifheit ein maximales Drehmoment bei hohem elektromechanischem Wirkungsgrad zu generieren, wobei Rastmomente und Welligkeit des Drehmoments zu vermeiden sind.

Bei der erfindungsgemäßen Pumpe wird dies wie folgt gelöst: Die Permanentmagnete sind durch die Unterbringung in beiden Rotorabdeckungen in zwei Ebenen geteilt, zwischen denen ein magnetischer Rückschluss über den Schaufelkanal besteht. Bei einer bevorzugten Ausführung des Rotors mit R = 20 mm, d = 1 mm und r₃ = 16 mm werden dann lediglich 5 g Magnetmasse (NdFeB) benötigt, mit entsprechend geringer Destabilisierung des Rotors bei axialer Auslenkung.

Erfindungsgemäß können die beiden Statoren um einen Betrag 1/3 bis 1/2 der Polteilung gegeneinander verdreht sein; also bei 6 Polen um 20 bis 30 Grad. Ebenso können die permanentmagnetischen Bereiche der beiden Rotorabdeckungen in einem Winkel von bis zu 1/6 der Polteilung gegeneinander verdreht sein, also um bis zu 10 Grad. Beide Maßnahmen tragen zur Unterdrückung von Rastmomenten und Welligkeit des Drehmoments bei.

Eine weiter Steigerung des elektromechanischen Wirkungsgrades, bei minimaler axialer Steifigkeit des Antriebs, kann durch eine optimierte Polüberdeckung der Statorzähne und der relativen Polüberdeckung der Permanentmagnete erfolgen.

Die radiale Stabilisierung der Rotorlage (Translation in x- und y-Richtung) wird durch die oben dargestellte Gestaltung des Rotors, der Rotorseitenräume, des zirkulären Ringspaltes sowie des strömungsabführenden Spiralkanals begünstigt, jedoch nicht bewirkt. Die radiale Zentrierung des Rotors erfolgt durch magnetische Reluktanzkräfte zwischen den permanentmagnetischen Bereichen im Rotor und den Kopfbereichen der Statorzähne. Im Betrieb der Pumpe wird die Stabilisierung durch die auf den Rotor wirkenden Kreiselkräfte weiter verstärkt.

## Patentansprüche

1. Zentrifugal-Pumpe, insbesondere für Blut in Herzersatz- oder -unterstützungseinrichtungen, mit einem innerhalb eines bis auf mindestens eine Einlass- und mindestens eine Auslassöffnung (12, 13) flüssigkeits- und gasdicht geschlossenen Gehäuses (11) lagerlos und drehbar angeordneten Pumpenrotor (14), der gleichzeitig der Rotor eines Antriebsmotors ist und einen oder mehrere über seinen Umfang gleichmäßig verteilte permanent magnetisierte Bereiche aufweist, wobei außerhalb des Gehäuses (11) oberhalb und unterhalb des Rotors (14) ein Stator (18, 19) des Antriebsmotors angeordnet ist, sodass die Statoren (18, 19) im Spalt zwischen sich und dem oder den permanent magnetisierten Bereichen des Rotors (14) einen rotierenden magnetischen Fluss erzeugen, wobei der Rotor (14) symmetrisch zu seiner Mittelebene ausgebildet ist und eine obere und untere Abdeckung (16, 17, 16', 17') aufweist **dadurch gekennzeichnet, dass** der Rotor (14) und/oder das Pumpengehäuse (11) derart geformt sind, dass sich die axialen Abstände zwischen der oberen und unteren Abdeckung (16, 17, 16', 17') und der oberen und unteren Gehäusewandung radial einwärts kontinuierlich verringern, derart, dass im radial inneren Bereich des Rotors (14) die Rotorseitenräume (26, 26', 27, 27') je einen Drosselspalt (116, 117) aufweisen, welche im Betrieb die radial nach innen gerichteten Rückströmungen in den Rotorseitenräumen (26, 26', 27, 27') derart beeinflussen, dass bei einer axialen Auslenkung des Rotors (14) oberhalb und unterhalb des Rotors (14) unterschiedliche Druckverteilungen entstehen, wodurch auf die überwiegende Fläche der Abdeckungen (16, 16', 17, 17') wirkende Kräfte erzeugt werden, die eine axiale Stabilisierung des Rotors (14) bewirken, und in gleicher Weise gegen ein Verkippen des Rotors (14) im Gehäuse (11) wirksam sind.

2. Zentrifugal-Pumpe nach Anspruch 1, **dadurch gekennzeichnet, dass** die radiale Zentrierung des Rotors (14) passiv durch Reluktanzkräfte erfolgt.

3. Zentrifugal-Pumpe nach einem der vorhergehenden Ansprüche, **dadurch gekennzeichnet, dass** der Rotor (14) vollständig aus para- und/oder ferromagnetischen Material gefertigt ist.

4. Zentrifugal-Pumpe nach einem der vorhergehenden Ansprüche, **dadurch gekennzeichnet, dass** mindestens ihre in Flüssigkeitskontakt stehenden Oberflächen mit einer den Flüssigkeitseigenschaften angepassten Beschichtung versehen sind.

5. Zentrifugal-Pumpe nach einem der vorhergehenden Ansprüche, **dadurch gekennzeichnet, dass** sie eine Blutpumpe ist, die in den Körper implantierbar ist.

## Claims

1. Centrifugal pump, especially for blood in heart replacement or heart assist devices, with a pump rotor (14) arranged for rotation without bearings, inside a housing (11) which is closed so as to be liquid tight and gas tight except for at least one inlet and at least one outlet opening (12, 13), the pump rotor (14) being simultaneously the rotor of a drive motor and having one or a plurality of permanently magnetised regions evenly distributed along its circumference, there being arranged a stator (18, 19) of the drive motor exteriorly of the housing (11) above and below the rotor (14), so that the stators (18, 19) produce a rotating magnetic flux in the gap between them and the permanently magnetised region or regions of the rotor (14), the rotor (14) being formed symmetrically about its centre plane and having an upper and lower covering (16, 17, 16', 17'), **characterised in that** the rotor (14) and / or the pump housing (11) are formed such that the axial distances between the upper and lower covering (16, 17, 16', 17') and the upper and lower housing wall diminish continuously radially inwardly, such that in the radially inner region of the rotor (14) the rotor side chambers (26, 26' 27, 27') each have a throttle gap (116, 117), which, in operation, influence the radially inwardly directed back flows in the rotor side chambers (26, 26' 27, 27') such that upon axial deflection of the rotor (14) different pressure distributions come about above and below the rotor (14), whereby forces are produced which act upon the preponderant surface of the covering (16, 17, 16', 17') to effect axial stabilisation of the rotor (14) and in the same way counteract tilting of the rotor (14) in the housing (11).

2. Centrifugal pump according to claim 1, **characterised in that** the radial centration of the rotor (14) comes about passively by reason of reluctance forces.

3. Centrifugal pump according to one of the preceding claims, **characterised in that** the rotor (14) is made wholly of paramagnetic and / or ferromagnetic material.

4. Centrifugal pump according to one of the preceding claims, **characterised in that** at least its surfaces that come into contact with liquid are provided with a coating that is adapted to the characteristics of the liquid.

5. Centrifugal pump according to one of the preceding claims, **characterised in that** it is a blood pump which can be implanted in the body.

## Revendications

1. Pompe centrifuge, en particulier pour du sang dans des dispositifs de remplacement du coeur ou d'assistance cardiaque, comportant un rotor de pompe (14) agencé sans palier et de manière à pouvoir tourner à l'intérieur d'un carter (11) fermé de façon étanche aux gaz et aux liquides, à l'exception d'au moins une ouverture d'admission et au moins une ouverture d'évacuation (12, 13), ledit rotor étant simultanément le rotor d'un moteur d'entraînement et présentant une ou plusieurs zones magnétisées permanentes réparties uniformément sur son pourtour, un stator (18, 19) du moteur d'entraînement étant agencé à l'extérieur du carter (11) au-dessus et en dessous du rotor (14) de telle sorte que les stators (18, 19) produisent un flux magnétique tournant dans l'interstice situé entre eux-mêmes et la ou les zones magnétisées permanentes du rotor (14), le rotor (14) étant réalisé de façon symétrique par rapport à son plan médian et présentant un couvercle supérieur et inférieur (16, 17, 16', 17') **caractérisé en ce que** le rotor (14) et/ou le carter de pompe (11) sont réalisés de telle sorte que les écarts axiaux séparant les couvercles supérieur et inférieur (16, 17, 16', 17') des parois supérieure et inférieure du carter diminuent de façon continue radialement vers l'intérieur, de telle sorte que dans la zone radiale interne du rotor (14) les chambres latérales du rotor (26, 26', 27, 27') présentent chacune une fente d'étranglement (116, 117), qui, lors du fonctionnement, influencent les reflux orientés radialement vers l'intérieur dans les chambres latérales du rotor (26, 26', 27, 27') de telle façon que, en cas de déviation axiale du rotor (14), au-dessus et en dessous du rotor (14) il apparaît des répartitions de pression différentes, ce qui produit des forces actives sur les surfaces principales des couvercles (16, 16', 17, 17'), forces qui provoquent une stabilisation axiale du rotor (14) et agissent de la même façon contre un basculement du rotor (14) dans le carter (11).

2. Pompe centrifuge selon la revendication 1,
**caractérisée en ce que** le centrage radial du rotor (14) s'effectue passivement par des forces de reluctance.

3. Pompe centrifuge selon l'une des revendications précédentes,
**caractérisée en ce que** le rotor (14) est entièrement réalisé à partir d'un matériau para- et/ou ferromagnétique.

4. Pompe centrifuge selon l'une des revendications précédentes,
**caractérisée en ce qu'**au moins ses surfaces en contact avec du liquide sont pourvues d'un revêtement adapté aux propriétés du liquide.

5. Pompe centrifuge selon l'une des revendications précédentes,
**caractérisée en ce qu'**il s'agit d'une pompe à sang pouvant être implantée dans le corps.
